**Europäisches Patentamt**

**European Patent Office**

**Office européen des brevets**

(11) Veröffentlichungsnummer: **0 401 661 A1**

# EUROPÄISCHE PATENTANMELDUNG

(21) Anmeldenummer: **90110248.3**

(22) Anmeldetag: **30.05.90**

(51) Int. Cl.5: **C11D 3/386, C12N 9/96**

(30) Priorität: **08.06.89 DE 3918761**

(43) Veröffentlichungstag der Anmeldung:
**12.12.90 Patentblatt 90/50**

(84) Benannte Vertragsstaaten:
**GR**

(71) Anmelder: **Henkel Kommanditgesellschaft auf Aktien**
**Henkelstrasse 67**
**D-4000 Düsseldorf 13(DE)**

(72) Erfinder: **Weiss, Albrecht, Dr.**
**Forellenweg 37**
**D-4018 Langenfeld(DE)**
Erfinder: **Maurer, Karl-Heinz, Dr.**
**Graf-Engelbert-Strasse 20**
**D-5657 Haan(DE)**

(54) **Flüssiges Enzymkonzentrat.**

(57) Bei einem flüssigen Enzymkonzentrat auf Basis einer durch Fermentation gewonnenen und von der Zellmasse und zumindest anteilweise von löslichen Beimengungen befreiten Hydrolaselösungen mit einem Enzymgehalt von 30 000 bis 400 000 Hydrolaseeinheiten sollte die Stabilität verbessert werden. Dies gelingt dadurch, daß man das Flüssigkonzentrat wie folgt aufbaut:
- 1,5 bis 20 Gew.-% Hydrolase
- 20 - 40 Gew.-% Propylenglykol und
- 0,5 - 5 Gew.-% Borsäure und/oder deren lösliche Salze
sowie gewünschtenfalls:
- 0,5 bis 5 Gew.-% Salze von 1 bis 3 basischen Carbonsäuren mit bis zu 6 C-Atomen.

EP 0 401 661 A1

## Flüssiges Enzymkonzentrat

Die Erfindung betrifft ein flüssiges Enzymkonzentrat, das als Rohstofflösung zur Herstellung von Flüssigwaschmitteln oder dergleichen verwendet werden kann.

Es ist bekannt, in Waschmitteln, auch Flüssigwaschmitteln, Enzyme, insbesondere Proteasen, einzusetzen. Für den Einsatz in festen Waschmitteln werden die Enzyme zu festen körnigen Zubereitungen aufgearbeitet. Mit dem Voranschreiten der Flüssigwaschmittel besteht ein Bedarf auch für derartige Zubereitungen, lagerfähige Stammlösungen herstellen zu können, die dann in einem nachfolgenden Verarbeitungsschritt dem Flüssigwaschmittel zugesetzt werden können.

Enzymlösungen von Hydrolasen, insbesondere Proteasen, sind jedoch per se nicht lagerstabil. Dies gilt insbesondere für solche Enzymlösungen, die für Flüssigwaschmittel eingesetzt werden sollen, da bei der Aufreinigung der Enzymlösungen anscheinend neben Verunreinigungen auch Stoffe entfernt werden, die eine Stabilisierung bedingen.

Der Fachliteratur sind bereits eine Reihe von Vorschlägen zu entnehmen, wie Enzyme in Flüssigwaschmitteln stabilisiert werden sollen. Da in Flüssigwaschmitteln jedoch der Enzymgehalt, verglichen mit anderen Stoffen, wie z.B. Tensiden, sehr gering ist und damit völlig andere Bedingungen herrschen, lassen sich diese Ergebnisse nicht auf Enzymkonzentrate übertragen.

So wird gemäß US-Patent 3,781,212 ein Flüssigwaschmittel vorgeschlagen, in dem zur Stabilisierung des Enzyms ein Polyol zusammen mit Borsäure verwendet wird. Dabei soll des Verhältnis Polyol : Borsäure kleiner 1 sein, d.h. Borsäure wird, verglichen mit dem Polyol im Überschuß, verwendet.

In der deutschen Offenlegungsschrift DE 31 25 533 wird ein wäßriges builderhaltiges flüssiges Waschmittel mit Enzymen, waschaktiven Substanzen, einem Polyol und Borsäure oder an deren Salzen in einem Gewichtsverhältnis Polyol : Borsäure von mehr als 1 beschrieben, wobei weiterhin ein Teil vernetztes Polyacrylat zugegen ist. Genannt werden neben anderen Polyolen auch 1,2-Propandiol und neben anderen Borverbindungen auch Borsäure und deren Salze. Vorgeschrieben wird, daß die Menge des Polyols 100 bis 200 Gew.-% der Menge an Borsäure betragen soll. Bevorzugt soll sie 100 bis 160 Gew.-% der Borsäuremenge betragen. Der Hydrolaseanteil von Flüssigwaschmittel beträgt üblicherweise jedoch nur um 0,1 Gew.-% Protein, bestenfalls bis zu 0,5 Gew.-%.

Aus der genannten deutschen Patentanmeldung lassen sich keine Rückschlüsse gewinnen, wie ein stabiles Enzymflüssigkonzentrat erhalten werden kann. Es ist daher Aufgabe der Erfindung, ein derartiges Flüssigkonzentrat bereitzustellen.

Gegenstand der Erfindung ist ein flüssiges Enzymkonzentrat auf Basis einer durch Fermentation gewonnenen von der Zellmasse und zumindest anteilsweise von löslichen Beimengungen befreiten Hydrolaselösung mit einem Enzymgehalt von 30 000 bis 400 000 Hydrolaseeinheiten enthaltend:
- 1,5 bis 20 Gew.-% Hydrolase
- 20 - 40 Gew.-% Propylenglykol und
- 0,5 - 5 Gew.-% Borsäure und/oder deren lösliche Salze
sowie gewünschtenfalls:
- 0,5 bis 5 Gew.-% Salze von 1 bis 3 basischen Carbonsäuren mit bis zu 6 C-Atomen

Die erfindungsgemäßen Enzymflüssigkonzentrate sind in einer bevorzugten Ausführungsform der Erfindung aufkonzentrierte, durch Fermentation gewonnene und zumindest anteilsweise gereinigte Enzymlösungen, die außer den erfindungsgemäß eingesetzten Stabilisierungsmitteln und den bei der Fermentation vorhandenen oder entstandenen Stoffen keine weiteren Bestandteile enthalten.

Als Stabilisierungsmittel wird erfindungsgemäß eine Kombination von Propylenglykol und Borsäure und/oder ihren Salzen eingesetzt. Die Menge an Propylenglykol (1,2-Propandiol) beträgt 20 bis 40 Gew.-% insbesondere 20 bis 30 Gew.-%. Die Borsäuremenge beträgt 0,5 bis 5 Gew.-%, insbesondere 1 bis 3 Gew.-%.

Die Gew.-%-Angaben bezüglich der Borsäure beziehen sich auf die Säure, auch wenn sie in der Lösung deren Salze vorliegen und demzufolge höhere Gew.-Mengen vorhanden sind.

Als Salze von 1 bis 3 basischen Carbonsäuren mit bis zu 6 C-Atomen können die löslichen Alkalimetall- oder Ammoniumsalze der entsprechenden Carbonsäuren eingesetzt werden. So können Salze von Ameisensäure, Propionsäure, Malonsäure, Maleinsäure oder Fumarsäure hier mitverwendet werden. Übliche Konzentrationen der Salze bezogen auf Gesamtzubereitung liegen zwischen 0,5 und 5 Gew.-%. So wurden beispielsweise günstige Ergebnisse mit 1 Gew.-% Ameisensäure oder 1 Gew.-% Maleinsäure, jeweils als Natriumsalz eingesetzt, erzielt.

Da eine Reihe von Hydrolaselösungen bei bestimmten pH-Werten instabil wird, ist es bevorzugt, die Borsäure als Salz einzusetzen, d.h. die Enzymlösungen vor, nach oder während der Zugabe der Borsäure auf einen pH-Wert zwischen 6 und 8 einzustellen. Geeignete Borsäuresalze sind die Alkalimetall- oder Ammoniumsalze, insbesondere das Natrium-oder Kaliumsalz.

Als Ausgangsenzymlösung können erfindungsgemäß Enzymlösungen eingesetzt werden, wie sie bei der Fermentation von Hefe oder Bakterien entstehen, die zur Bildung von Hydrolasen befähigt sind. Bevorzugt sind Lösungen, die als Hydrolasen, Proteasen, insbesondere Subtilisin Proteasen, enthalten. Unter diesen werden solche auf Basis von Subtilisin Carlsberg oder Subtilisin BBN' bevorzugt.

Zur Erzeugung der erfindungsgemäßen Enzymkonzentrate werden aus den Fermenterlösungen die Zellanteile abgetrennt und dann die Enzyme angereichert. Dies kann beispielsweise durch eine Kombination von Mikro- und Ultrafiltration oder auch durch Fällungs- und Flockungsstufen mit gewünschtenfalls nachträglichem Eindampfen der wäßrigen Lösung durchgeführt werden.

Den so erhaltenen nachgereinigten Enzymlösungen wird 1,2-Propandiol und die Borsäure zugesetzt. Um einen kritischen pH, der bei manchen Hydrolasen bereits bei 4 liegt, nicht zu unterschreiten, kann vor oder während der Zugabe der Borsäure mit Natronlauge auf den gewünschtne pH zwischen 6 und 8 eingestellt werden.

Die erfindungsgemäßen Zubereitungen können darüber hinaus noch Konservierungsmittel enthalten. In vielen Fällen ist dies doch nicht nötig, da bereits die Kombination aus Propylenglykol und Borsäure konservierend wirkt. Ein geeignetes Konservierungsmittel ist beispielsweise Formaldehyd.

Die Erfindung beruht auf dem synergistischen Stabilisierungseffekt von Borsäure und Propylenglykol auf derartige Enzyme, der innerhalb der angegebenen Grenzen besonders günstige Stabilitätswerte liefert, wie dies in den nachfolgenden Beispielen ersichtlich ist.

## Beispiele

Beispiel 1:

Die Lösung einer Subtilisin Carlsberg Protease mit einer Aktivität von 130 EP-Einheiten /g wurde mit Mengen von 0, 20, 30 und 50 Gew.-% Propylenglykol inkubiert und bei 60°C einem beschleunigten Alterungstest unterzogen. Nach 24 Stunden wurden die folgenden Restaktivitäten gemessen:

| ohne Propylenglykol | 20 Gew.-% Propylenglykol | 30 Gew.-% Propylenglykol | 50 Gew.-% Propylenglykol |
|---|---|---|---|
| kleiner 5 | 23 | 25 | 16 |

Beispiel 2:

Der Versuch wurde wiederholt, wobei bei 20 Gew.-% Propylenglykol zusätzlich 1, 3 und 5 Gew.-% Natriummetaborat zugegeben wurden. Nach 24 Studen wurden folgende Restaktivitäten in Prozent gemessen:
1 % Metaborat 50 %
3 % Metaborat 46 %
5 % Metaborat 34 %.

Beispiel 3:

Der Versuch wurde wiederholt, wobei eingesetzt wurden:
1 Gew.-% Natriummetaborat
30 Gew.-% Propylenglykol und
1 Gew.-% Ameisensäure bzw. Maleinsäure
Nach 100 Minuten Lagerung bei 60 °C wurde kein Nachlassen der Aktivität beobachtet, wohingegen ohne die Zugabe der Carbonsäuresalze die Aktivität auf 84 % nachließ.

Die Messung der Enzymaktivität erfolgte in allen Fällen nach der sogenannten EPE-Methode, beschrieben in der deutschen Patentanmeldung DE 37 34 047. Das Meßprinzip ist die Spaltung einer phenolischen Esterbindung von N-CBZ-Aminosäure-p-Nitrophenylester bei 25 °C und einem pH von 6.0, wobei die Konzentration des entstehenden p-Nitrophenols photometrisch bei 340 nm bestimmt wird.

## Ansprüche

1. Flüssiges Enzymkonzentrat auf Basis einer durch Fermentation gewonnenen von der Zellmasse und zumindest anteilsweise von löslichen Beimengungen befreiten Hydrolaselösung mit einem Enzymgehalt von 30 000 bis 400 000 Hydrolaseeinheiten enthaltend
- 1,5 bis 20 Gew.-% Hydrolase
- 20 - 40 Gew.-% Propylenglykol und
- 0,5 - 5 Gew.-% Borsäure und/oder deren lösliche Salze
sowie gewünschtenfalls:
- 0,5 bis 5 Gew.-% Salze von 1 bis 3 basischen Carbonsäuren mit bis zu 6 C-Atomen

2. Enzymflüssigkonzentrat nach Anspruch 1, dadurch gekennzeichnet, daß als Hydrolasen Proteasen, insbesondere Subtilisin Proteasen, enthalten sind.

3. Enzymflüssigkonzentrat nach einem der Ansprüche 1 oder 2, dadurch gekennzeichnet, daß Propylenglykol in Mengen von 20 bis 30 Gew.-%, bezogen auf Enzymflüssigkonzentrat, vorhanden ist.

4. Flüssiges Enzymkonzentrat nach einem der Ansprüche 1 bis 3, dadurch gekennzeichnet, daß Borsäure und/oder ihre Salze in Mengen von 1 bis 3 Gew.-% (berechnet als Borsäure) und bezogen auf Enzymflüssigkonzentrat vorhanden ist.

5. Enzymflüssigkonzentrat nach einem der Ansprüche 1 bis 4, dadurch gekennzeichnet, daß die Borsäure als Natrium-, Kalium- oder Ammoniumsalz vorliegt.

6. Flüssiges Enzymkonzentrat nach einem der Ansprüche 1 bis 5, dadurch gekennzeichnet, daß es einen pH-Wert zwischen 6 und 8 aufweist.

4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | GB-A-2140819 (COLGATE-PALMOLIVE) <br> * Seite 1, Zeile 63 - Seite 2, Zeile 4; Ansprüche 1-10 * | 1-8 | C11D3/386 <br> C12N9/96 |
| A | EP-A-80223 (UNILEVER) <br> * Seite 5; Anspruch 1 * | 1 | |
| A | GB-A-2126242 (COLGATE-PALMOLIVE) <br> * Ansprüche 1-9, 30 * | 1 | |

**RECHERCHIERTE SACHGEBIETE (Int. Cl.5 )**

C11D
C12N

Der vorliegende Recherchenbericht wurde für alle Patentansprüche erstellt

| Recherchenort | Abschlußdatum der Recherche | Prüfer |
|---|---|---|
| DEN HAAG | 10 AUGUST 1990 | PFANNENSTEIN H. |